# EUROPEAN PATENT SPECIFICATION

(11) **EP 0 458 417 B1**
(45) Date of publication and mention of the grant of the patent: **17.04.1996**
(21) Application number: 91201204.4
(22) Date of filing: 17.05.1991
(51) Int. Cl.: C07C 39/17, C07C 37/14, C08G 8/30, C08G 59/08, C08G 59/62, C08G 8/20, C08G 8/12, C08G 59/06, C08G 59/22, C08L 63/02, C08L 61/04, C08L 61/12

(54) **Adducts of phenolic compounds and cyclic terpenes and derivatives of said adducts**
Addukte phenolischer Verbindungen und cyclische Terpene und Derivate dieser Addukte
Adduits à base de composés chimiques phénoliques et de terpènes cycliques et dérivés de ces adduits

(30) Priority: 23.05.1990 JP 131416/90; 13.06.1990 JP 152845/90; 25.06.1990 JP 164179/90
(43) Date of publication of application: 27.11.1991
(73) Proprietor: SHELL INTERNATIONALE RESEARCH MAATSCHAPPIJ B.V., NL-2596 HR Den Haag (NL)
(72) Inventor: Murata, Yasuyuki, c/o Yuka Shell Epoxy K.K., Minato-ku, Tokyo 106 (JP); Tanaka, Ryohei, c/o Yuka Shell Epoxy K.K., Minato-ku, Tokyo 106 (JP); Kurio, Takuya, c/o Yuka Shell Epoxy K.K., Minato-ku, Tokyo 106 (JP); Nakanishi, Yoshinori, c/o Yuka Shell Epoxy K.K., Minato-ku, Tokyo 106 (JP); Goto, Fumio, Fuchu-city, Hiroshima Pref. (JP); Minematsu, Wasaku, Fuchu-city, Hiroshima Pref. (JP)

(56) References cited:
- DE-A- 2 616 592
- DE-B- 2 552 175
- DE-C- 712 701
- FR-A- 792 623
- FR-A- 1 323 858
- US-A- 3 692 844
- PATENT ABSTRACTS OF JAPAN, vol. 4, no. 140, (C-26)1980; & JP-A-55 89 318
- PATENT ABSTRACTS OF JAPAN, vol. 4, no. 151, (C-28)1980; & JP-A-55 94 921

## Description

The present invention is concerned with adducts of phenolic compounds and cyclic terpenes, with condensation products of said adducts and an aldehyde or ketone, with glycidyl ethers of said condensation products and with the use of said condensation products or said glycidyl ethers in the production of thermoset compositions.

Epoxy resins are widely utilized as thermosetting resins in a variety of fields, such as laminating, painting, adhesion, sealing, encapsulating, casting and the like.

In recent years, more severe demands have been set for the macromolecule epoxy resins used for micro-encapsulating electronic equipment and the epoxy resins currently used in the market do not meet such higher demands. For instance, an epoxy resin composition which is cured with a novolak resin, as now applied to the field of micro-encapsulating semiconductors, frequently fail advanced tests for high thermal resistance and low hygroscopicity. The high integration technology of semiconductor devices had led to miniaturization and thinning of the components. Furthermore, the mountings of semiconductor devices are now switching over to surface mountings, wherein the devices have to be directly immersed in a bath of molten solder. They are thus exposed to high temperatures thereby expanding moisture absorbed in the encapsulating resin to cause cracking taking place.

Consequently, high thermal resistance and low hygroscopicity are required at the same time for cracking resistant encapsulating material, and the presently available encapsulating material comprising cresol novolak-type epoxy resin and, as a curing agent, phenol novolak-type resin is no longer satisfactory in view of poor thermal resistance and hygroscopicity. Other requirements for encapsulating materials are the need to obtain high glass transition temperature and reduced internal stress.

In e.g. FR-A 1 323 858 adducts obtained by reacting a mono- or dihydric phenolic compound with a cyclic terpene, are described.

The cyclic terpene compound, which is the raw material for preparing said cyclic terpene-skeleton-containing polyhydric phenol compound, may be a monocyclic terpene compound or a bicyclic terpene compound, and may typically be exemplified as follows:
Limonene: 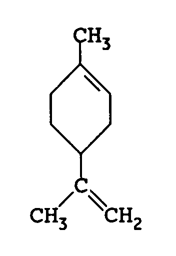
Dipentine:
Being an optical isomer of limonene:
Terpinolene: 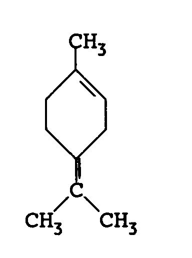
Pinene: 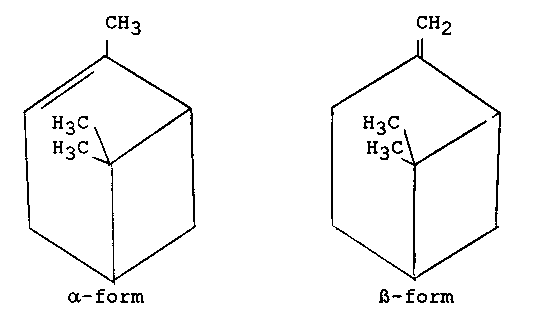
Terpinene: 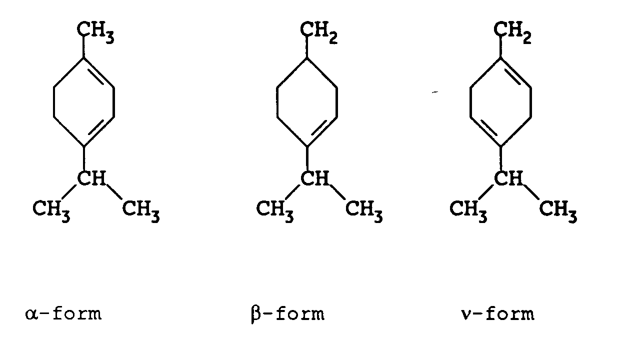
Menthadiene: 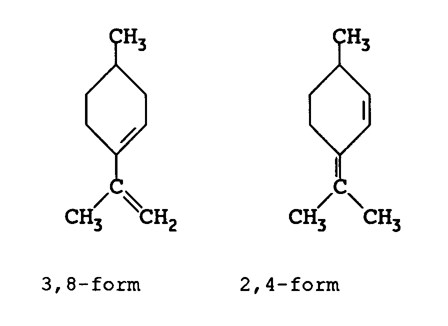
Preferred cyclic terpenes are limonene and the terpinenes.

The mono- or dihydric phenolic compounds, the other starting material for preparing said adducts, may be exemplified by phenol, cresol, xylenol, propylphenol, nonylphenol, hydroquinone, resorcinol, methoxyphenol, bromophenol, bisphenol A, bisphenol F and the like. Phenol is the preferred reactant.

The addition reaction between said cyclic terpene compounds and said phenolic compounds may be performed by reacting one mol of the cyclic terpene compounds for 1 to 12, preferably 2 to 8 mol, of the phenolic compound. The presence of an acid catalyst is recommended and the temperature should range from 40 °C to 160 °C. Said acid catalysts are exemplified by hydrochloric acid, sulphuric acid, phosphoric acid, polyphosphoric acid, boron tetrafluoride or its complex, activated clay and the like. In the reaction, solvent may or may not be employed but solvent of aromatic hydrocarbons, alcohols, ethers and the like may usually be used. The reaction should be continued long enough to prepare adducts comprising at least 1.5, preferably 2 phenolic moieties per cyclic terpene moiety. Reacting for 1.5 to 8 hours is usually adequate for achieving that purpose. The adducts prepared in this manner from limonene and phenol, respectively from α-terpinene and phenol are represented by the following formulae:

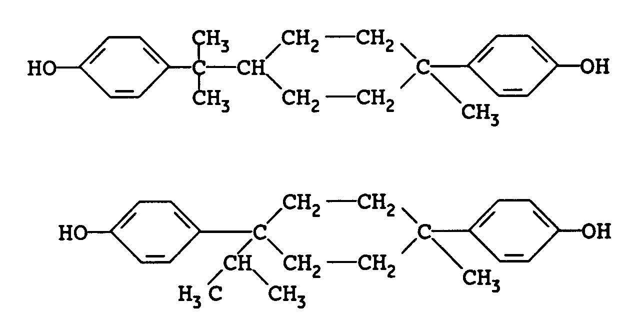

It is known in the art, e.g. from FR-A 1 323 858 and DE-A 26 16 592, to convert the adducts into resins by condensing the aforesaid adducts with an aldehyde or ketone, in particular formaldehyde.

Suitable aldehydes or ketones used in the condensation reaction with the aforesaid adducts are formaldehyde, acetaldehyde, benzaldehyde, hydroxy benzaldehyde, acetone, cyclohexanone and the like. Formaldehyde is preferred.

The molar ratio of the phenol compounds to the aldehydes and/or ketones in the condensation reaction is one mol of the adduct to 0.1-1.0, preferably 0.2-0.7 mol of the aldehydes or ketones, and the reaction is preferably performed in the presence of an acidic catalyst at a temperature ranging from 40 °C to 200 °C for 1-12 hours.

The acidic catalyst used in the condensation reaction may be chosen from: mineral acids such as hydrochloric acid, sulphuric acid and the like, organic acids such as oxalic acid, toluene sulphonic acid and the like, and organic acid salts. The amount of the acidic catalyst used in the reaction is 0.5-5 parts by weight for 100 parts by weight of the adducts. In the condensation reaction, an inert solvent such as aromatic hydrocarbons, alcohols, ethers or the like may be used.

FR-A 1 323 858 describes that, for a phenol-terpene adduct to be suitable in the preparation of a polyepoxy resin, the adduct must have a hydroxyl content of 7.5 to 8.0% and a molecular weight of 330 to 360. This implies that the suitable adducts must consist of about two phenol moieties and one terpene moiety. If these requirements are not fulfilled the products cause problems in the formation of polyepoxy resins.

It has now been found that reaction products of an epohalohydrin with a condensate of a phenol-terpene adduct do not cause any problems. Moreover, cured products of the glycidyl ether resins, thus obtained, have a high thermal resistance and a low hygroscopicity.

Accordingly, the present invention provides a glycidyl ether resin obtained by reacting a product obtained by condensing an adduct of a mono- or dihydric phenolic compound and a cyclic terpene with an aldehyde or ketone, with an epihalohydrin.

At first, said condensation products are dissolved in epihalohydrins of 2-20 molar equivalent for one molar equivalent of the phenolic hydroxy group of said condensates. Then, the solution is allowed to react by addition of an alkali metal hydroxide in a solid or in an aqueous solution of 1-2 molar equivalent for one molar equivalent of the phenolic hydroxy group whilst stirring the reaction mixture.

This reaction may be performed under atmospheric pressure or reduced pressure, and the temperature may be from 60 to 105 °C in the reaction under atmospheric pressure and from 50-80 °C in the reaction under reduced pressure. The reaction may optionally be performed in a manner of dehydrating the reaction system, by azeotropically distilling the reaction solution while maintaining a certain predetermined temperature, by cooling the evaporated vapour to give a condensate, by separating an oily component from aqueous component in said condensate and by returning the oily component separated from the aqueous component to the reaction system. The addition of the alkali metal hydroxide should be performed intermittently or continuously for a time period of 1 to 8 hours in order to suppress an abrupt reaction.

After completion of the reaction, the by-produced insoluble salts are removed by filtration or by washing with water, and then the unreacted epihalohydrin is removed by distilling under reduced pressure to give the envisaged epoxy compounds.

In the reaction, epichlorohydrin or epibromohydrin is usually used as said epihalohydrins and NaOH or KOH is usually used as said alkali metal hydroxides.

Moreover, a catalyst comprising a quaternary ammonium salt such as tetramethyl ammonium chloride, tetraethyl ammonium bromide and the like; or a tertiary amine such as benzyldimethylamine, 2,4,6-tris-(dimethylaminomethyl)phenol and the like; or an imidazole such as 2-ethyl-4-methylimidazole, 2-phenylimidazole and the like; or a phosphonium salt such ethyltriphenylphosphonium iodide and the like may be employed in this reaction. Furthermore, an inert organic solvent of alcohols such as ethanol, isopropanol and the like; of ketones such as acetone, methyl ethyl ketone and the like; of ethers such as dioxane, ethyleneglycol dimethylether and the like; and of aprotic polar solvents such as dimethyl sulphoxide, dimethyl formamide and the like may be employed in this reaction.

The glycidyl ether resins so obtained are compositions which may be employed as such or in admixture with other epoxy compounds, or bisphenols such as bisphenol A or F, resorcinol, hydroquinone, phenol novolak, cresol novolak and bisphenol A novolak.

The glycidyl ether resins of the present invention will usually be reacted with a curing agent and, if necessary, they may further include a curing accelerator, inorganic fillers, coupling agents, fire retardants, plasticizers, organic solvents, reactive diluents, pigments, antistatic agents, glass fibre reinforcement and the like. As curing agents, any known curing agent generally used for epoxy resins may be used. Novolak-type phenol resins, novolak-type cresol resins, polyfunctional phenol resins obtained by a condensation reaction between various phenols and various aldehydes such as hydroxy benzaldehyde, croton aldehyde, glyoxal, acid anhydrides such as tetrahydrophthalic anhydride, and pyromellitic anhydride and amines such as diamino diphenyl methane, and diamino diphenylsulphone are suitable curing agents.

The curing accelerators may be imidazoles, such as 2-methylimidazole, and 2-ethyl-4-methylimidazole, amines such as 2,4,6-tris(dimethylaminomethyl)phenol, and benzylmethylamine, organic phosphor compounds such as tributylphosphine and triphenylphosphine. The inorganic fillers may be exemplified by silica, glass powder, alumina, zirconia and the like, and the fire retardants may be brominated epoxy resins, antimony trioxide, and phosphoric acid.

The cured products of the glycidyl ether resin compositions according to the present invention have a higher thermal resistance and a lower hygroscopicity as compared with the cured product of the conventional epoxy resin compositions. Thus the epoxy resin compositions of the present invention are able to be advantageously used in the fields of various encapsulating, laminating, moulding, adhesion and surface coating applications.

The aforesaid adducts and their condensates with aldehydes or ketones can be employed as components for preparing thermoset macromolecular compounds. The adducts are at least dihydric hydroxy compounds whilst the condensates, like novolak-phenol type resins, are polyhydric compounds. They can thus be employed as thermosetting resins to be cured with conventional curing agents such as di- or polybasic carboxylic acids or anhydrides thereof or they can be employed as curing agents, like phenol-novolak resins, or bisphenolic curing agents. When employed as curing agents for polyglycidyl ether derivatives of o-cresol-novolak type resins the resulting castings have, in combination, a high glass transition temperature (185 °C or more), an attractive flexural modulus (1420 kg.mm⁻² or more), a good flexural strength (15.1 kg.mm⁻² or more) and an excellent low moisture adsorption (0.73% by weight or even better).

Accordingly, in a further aspect this invention is concerned with a particular use of the aforesaid condensates namely as components of thermosetting compositions in admixture with polyglycidyl ether derivatives of an o-cresol-novolak type resin, or diglycidyl ethers of bishydroxy biphenyl compounds, preferably having the following formula: 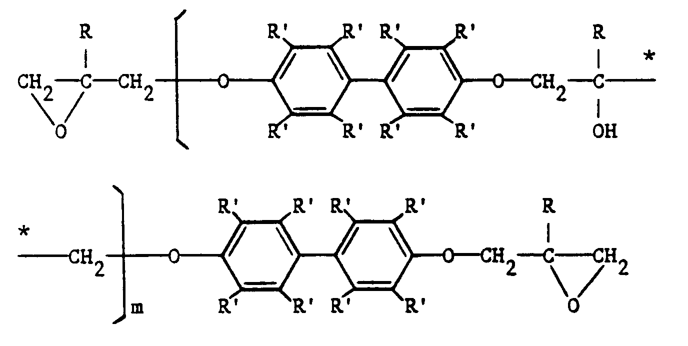
wherein R represents hydrogen atom or methyl group, R' represents hydrogen atom, methyl group, ethyl group, isopropyl group, phenyl group, chlorine atom or bromine atom with the proviso that the substituents R' may be the same or different from one another and m is a number with an average value of from 0 to 5. Of such biphenylic compounds the diglycidyl ether of 4,4'-dihydroxybiphenyl is the compound of choice. Other suitable diglycidyl ethers are those obtained by reacting
3,3',5,5'-tetramethyl-4,4'-dihydroxybiphenyl,
3,3',5,5'-tetraethyl-4,4'-dihydroxybiphenyl, and
3,3',5,5'-tetrapropyl-4,4'-dihydroxybiphenyl with epihalohydrins.

The thermosetting resin systems set out in the preceding paragraph are particularly useful in the micro-encapsulation of electronic parts: they have a high glass transition temperature, a good crack resistance in soldering bath and a markedly reduced internal stress.

### Example 1

A 5 l three-necked flask, equipped with a thermometer, a stirrer and a reflux condenser was filled with 3384 g (36 mol) of phenol and 34 g of a complex of boron trifluoride etherate, and, while maintaining the temperature at 70 to 80 °C, 816 g (6 mol) of limonene was added dropwise into the mixture during a time period of 3 hours. Stirring was continued for another 2 hours. After three times washing of the reaction product with distilled water and a removal of the unreacted phenol and by-product by distillation under reduced pressure, followed by a maintenance of the mixture at 5 mmHg and 160 °C for one hour, 1520 g of a dihydric cyclic terpene-skeleton-carrying phenol compound (adduct) was obtained. As a result of analysis by liquid chromatography, the purity of this phenol compound was found to be 89%.

Next, 1100 g of the cyclic terpene-skeleton-carrying phenol compound (adduct) prepared in the above-mentioned manner, were dissolved in 1000 g of toluene and 5 g of oxalic acid by raising the temperature to 80 °C. To this solution 75 g of aqueous formaldehyde (36%) were added dropwise during a time period of one hour while maintaining the temperature of the solution at 80 °C. The mixture was allowed to react at 80 °C for another hour. Thereafter, water and toluene were removed by vacuum-heating at 5 mmHg and 160 °C for one hour. The obtained macromolecularized polyhydric phenol compound (condensate) was a yellow solid with a softening point at 126 °C.

1020 g of the macromolecularized polyhydric phenol compound (condensate) prepared in the above-mentioned manner were reacted with 2775 g of epichlorohydrin and 1080 g of isopropyl alcohol which were mixed to form a solution and then heated to 35 °C. To the reaction mixture, 544 g of an aqueous solution of sodium hydroxide (48.5% by weight) was added dropwise during a time period of one hour, while the temperature of the reaction system was elevated gradually to 65 °C at the end of the dropwise addition. Thereafter, the mixture was allowed to react for 30 min. while being held at a temperature of 65 °C. After the reaction, the by-produced salts and the sodium hydroxide were removed from the reaction mixture by washing with water. Next, the excess (unreacted) epichlorohydrin and the isopropyl alcohol were removed by distillation under reduced pressure to yield a crude diglycidyl ether resin.

The crude polyglycidyl ether resin was then dissolved in 1300 g of toluene and combined with 30.0 g of an aqueous solution of sodium hydroxide (48.5% by weight). The combined solution was allowed to react at 65 °C for one hour. After the reaction, the reaction system sodium phosphate was added to neutralize the excess of sodium hydroxide. Then the product was washed with water to remove by-produced salt; the solvent was removed from the system under reduced pressure. The obtained polyglycidyl ether compound was a pale yellow solid with a softening point of 68 °C, it had an epoxy equivalent weight of 249.

### Example 2

A series of reactions and after treatments similar to those described in Example 1 were repeated except that 80 g of benzaldehyde was used in lieu of 75 g of the aqueous solution of formaldehyde (36%), and the amount of the macromolecularized polyhydric phenol used in the reaction with epichlorohydrin was altered to 1050 g, to give a polyglycidyl ether with a softening point at 71 °C and an epoxy equivalent weight of 252.

### Example 3

A reaction and after treatment similar to those described in Example 1 were repeated except that; 816 g (6 mol) of terpinene was used in lieu of the limonene to give 1480 g of a dihydric cyclic terpene-skeleton-carrying phenol compound with a purity of 86% determined by a liquid chromatographic analysis.

Next, a condensation with formaldehyde reaction was performed on the dihydric compound in a manner similar to that described in Example 1, to give 1050 g of a macromolecularized polyhydric phenol compound of yellow solid with a softening point at 121 °C. Then, 1020 g of a macromolecularized polyhydric phenol were reacted with 2775 g of epichlorohydrin in a manner similar to that described in Example 1, to give 1250 g of a polyglycidyl ether compound being a pale yellow solid with a softening point at 70 °C and an epoxy equivalent weight of 254.

The epoxy resins prepared as set out herein were evaluated in encapsulating formulations marked in Table I, all parts are parts by weight, 2 comparative resin systems were also evaluated.

Carnauba wax was used as mould releasing agent. Each set of ingredients was mixed on a roll at a temperature ranging from 90 °C to 110 °C during 5 min. and the molten mixture was poured out in a sheet form and after solidification, pulverized to give the respective material ready for moulding. Each of the compositions was subjected to low-pressure transfer moulding at a die temperature of 180 °C for a moulding time of 180 sec. to produce a test piece for determining hygroscopicity comprising a 44 pins flat plastic package (FPP) encapsulating a dammy tip. The mouldings were cured at 180 °C for 8 hours. As demonstrated in Table I, each of the materials of this invention gave an excellent cured product having a high glass-transition temperature and a low hygroscopicity and thus being suited for encapsulating semiconductor devices.

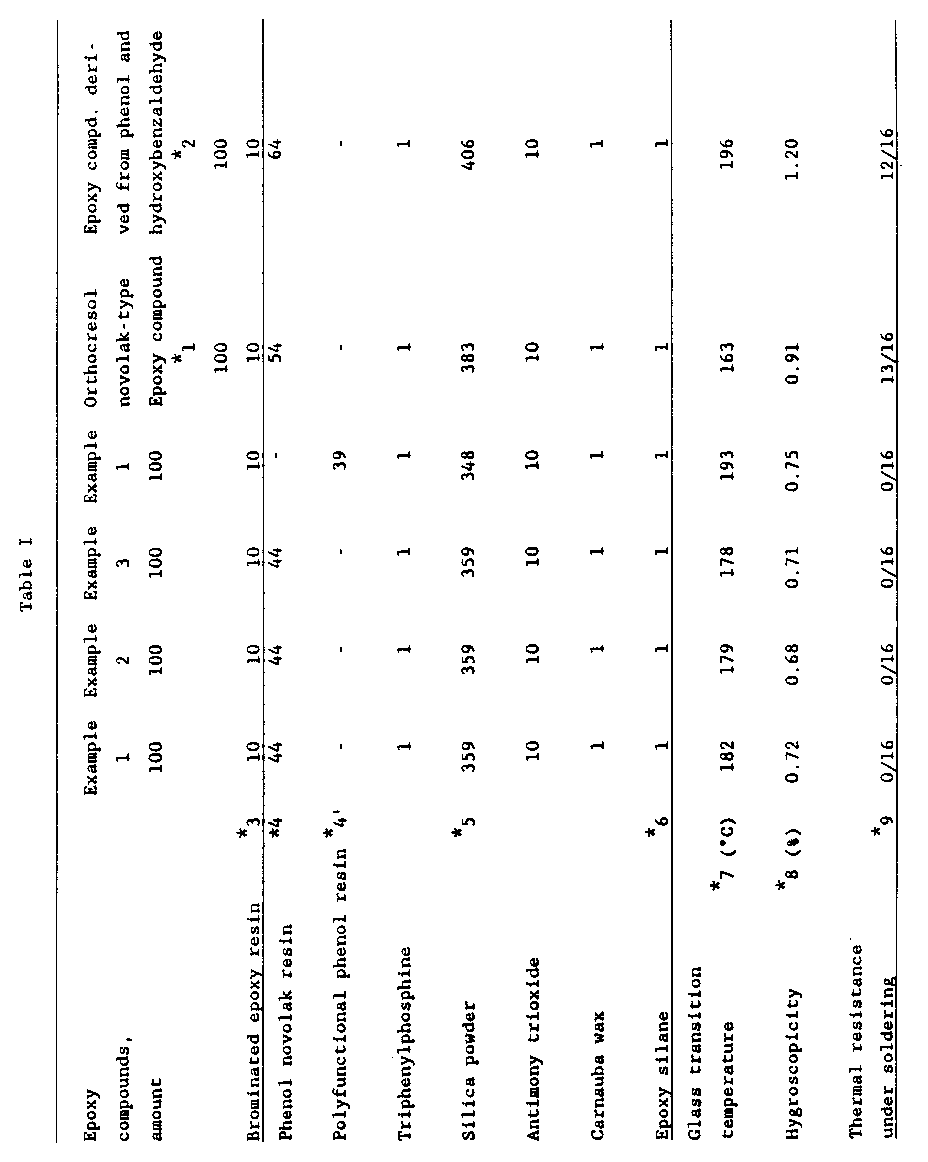

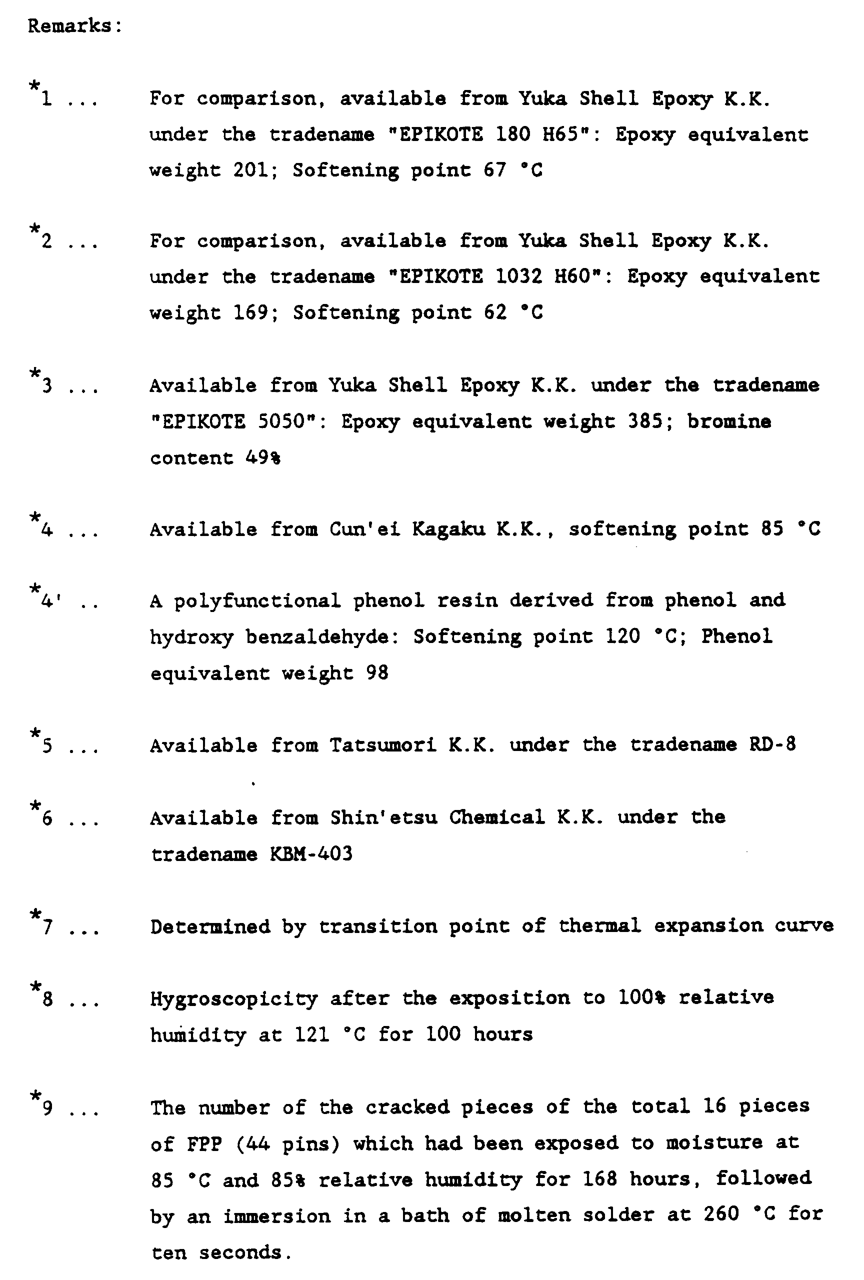

### Example 4

In a 5 l reactor equipped with a stirrer, a thermometer, and a reflux-condenser were mixed 242 g of 4,4'-bishydroxy-3,3',5,5'-tetramethyl biphenyl, 1295 g of epichlorohydrin, and 504 g of isopropyl alcohol. The resulting solution was heated to 35 °C, followed by dropwise addition, for one hour, of 190 g of an aqueous solution of sodium hydroxide (48.5% by weight). During the dropwise addition, the temperature of the solution gradually lose until the reaction mixture reached 65 °C at the end of the dropwise addition. The reaction was continued at 65 °C for 30 minutes.

The reaction product was washed with water to remove the by-product salts and excess of sodium hydroxide. Then, the reaction product was further purified by distillation under reduced pressure, to obtain a crude epoxy compound. The thus obtained crude diglycidyl ether compound was dissolved in 596 g of toluene, followed by 12.9 g of an aqueous solution of sodium hydroxide (48.5% by weight) being added thereto for effecting a reaction at 65 °C for one hour. Sodium primary phosphate was added to neutralize the excess of sodium hydroxide, followed by water-washing for removing the by-product salts.

The diglycidyl ether thus obtained was completely liberated from solvent by heating under reduced pressure. The thus obtained purified diglycidyl ether compound had the general formula marked hereinbefore, wherein polymerization degree (m) and epoxy equivalent weight were 0.1 and 185 respectively. This epoxy resin will be referred to as "Epoxy Resin a1" hereinafter.

### Example 5

In place of 4,4'-bishydroxy-3,3',5,5'-tetramethylbiphenyl employed in example 4, use was made of 186 g of 4,4'-bishydroxybiphenyl but, in all other respects of the same recipe as that followed in example 4.

The thus obtained diglycidyl ether resin had the general marked hereinbefore wherein polymerization degree (m) and epoxy equivalent weight were 0.1 and 157 respectively. This epoxy resin will be referred to as "Epoxy Resin a2" hereinafter.

Epoxy resins a1 and a2 were evaluated in formulations comprising the phenol-limonene adduct described in example 1, the phenol-limonene-formaldehyde condensate described in example 1 and the phenol-limonene-benzaldehyde condensate described in example 2.

The testing of the formulations was identical to the methods described in example 3. Table II, marking parts by weight, shows the results so produced.

The respective epoxy resin compositions obtained with the epoxy resins of this invention were low both in modulus of elasticity and hygroscopicity as compared to the prior art formulations. They have excellent crack resistance under soldering and high volume resistivity with excellent electrical characteristics.

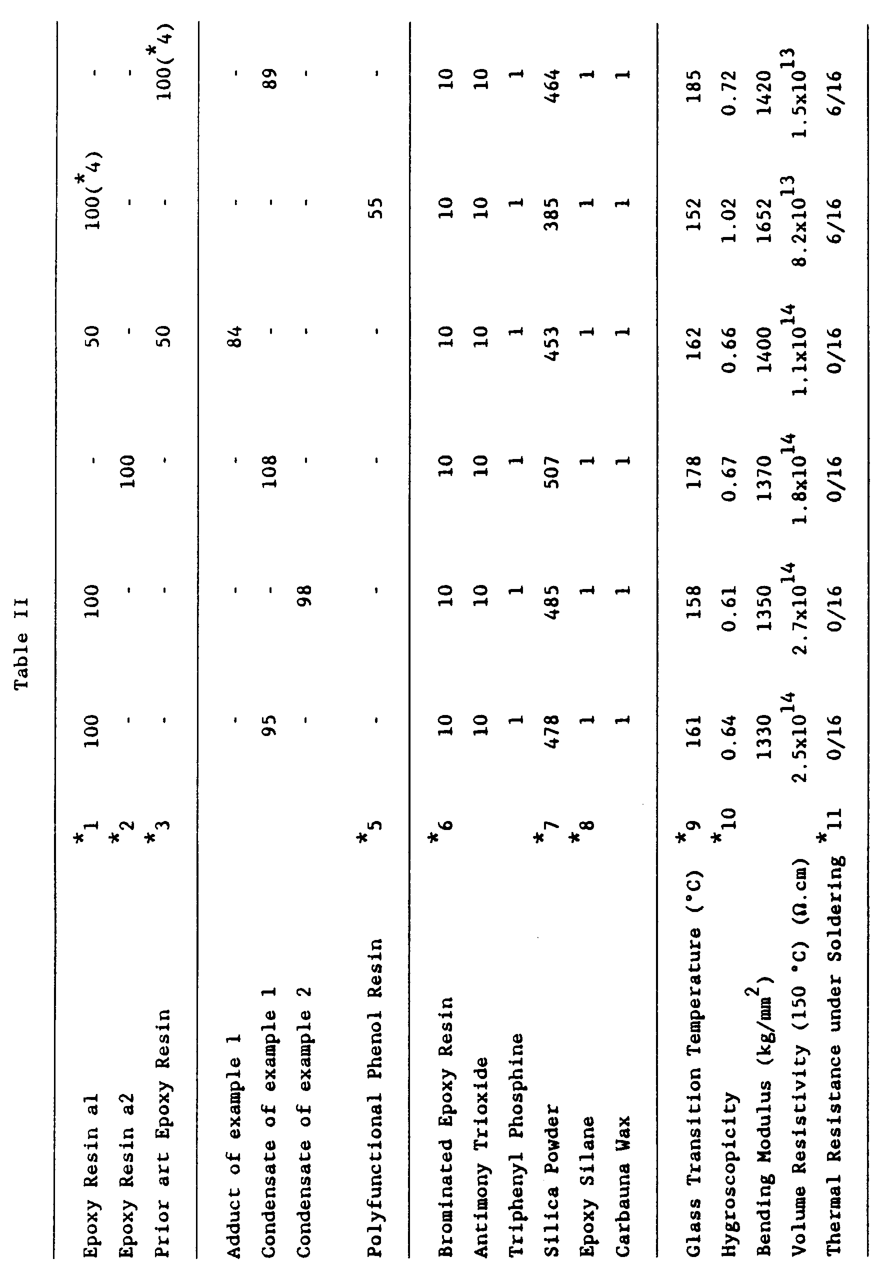

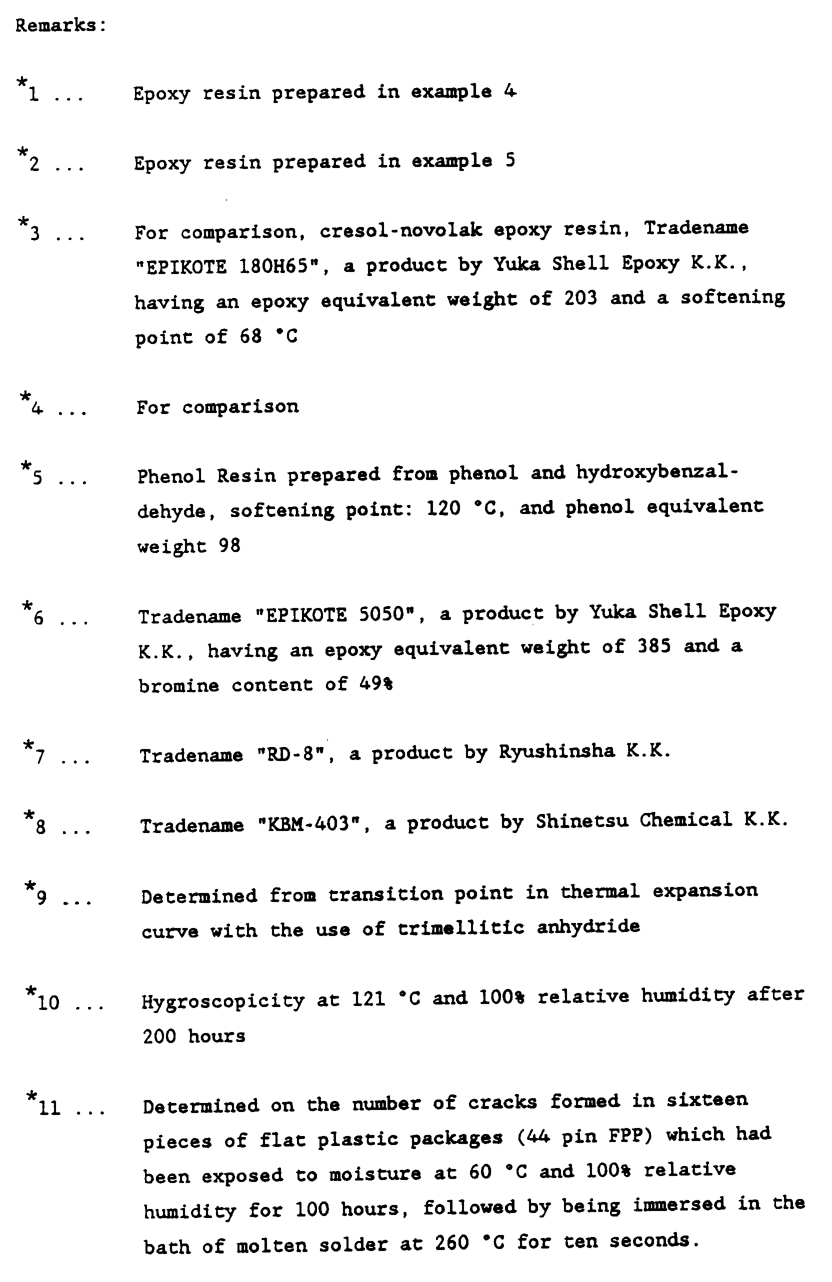

## Claims (Claims for the following Contracting State(s): BE, CH, DE, FR, GB, IT, LI, NL)

1. A glycidyl ether resin obtained by reacting a product obtained by condensing an adduct of a mono- or dihydric phenolic compound and a cyclic terpene with an aldehyde or ketone, with an epihalohydrin.

2. A glycidyl ether resin according to claim 1, in which the epihalohydrin is epichlorohydrin.

3. A thermosetting resin composition comprising as curing agent a reaction product obtained by condensing an adduct of a mono- or dihydric phenolic compound and a cyclic terpene with an aldehyde or ketone, and a diglycidyl ether of a bishydroxybiphenyl compound, or a polyglycidyl ether derivative of an o-cresol-novolak type resin.

4. A composition according to claim 3, in which the diglycidyl ether has the formula: 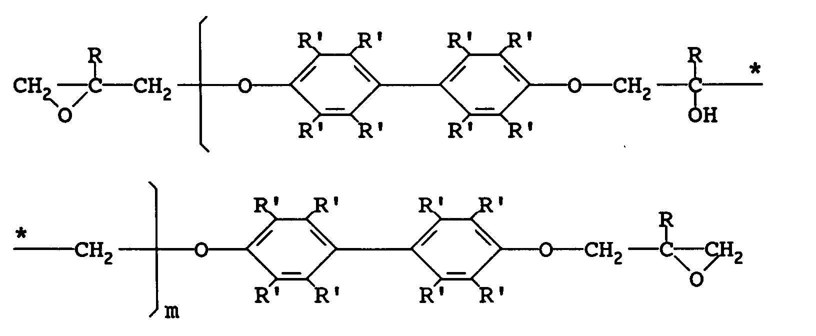 wherein R represents hydrogen atom or methyl group, R' represents hydrogen atom, methyl group, ethyl group, isopropyl group, phenyl group, chlorine atom or bromine atom with the proviso that the substituents R' may be the same of different from one another and m is a number with an average value of from 0 to 5.

## Claims (Claims for the following Contracting State(s): ES)

1. A process for the preparation of a glycidyl ether resin by reacting a product obtained by condensing an adduct of a mono- or dihydric phenolic compound and a cyclic terpene with an aldehyde or ketone, with an epihalohydrin.

2. A process according to claim 1, in which the epihalohydrin is epichlorohydrin.

3. A process for the preparation of a thermosetting resin composition by mixing together a reaction product obtained by condensing an adduct of a mono- or dihydric phenolic compound and a cyclic terpene with an aldehyde or ketone, and a diglycidyl ether of a bishydroxybiphenyl compound, or a polyglycidyl ether derivative of an o-cresol-novolak type resin.

4. A process according to claim 3, in which the diglycidyl ether has the formula: 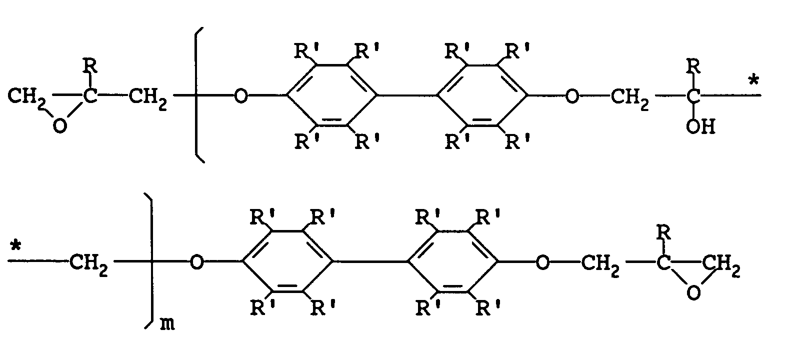 wherein R represents hydrogen atom or methyl group, R' represents hydrogen atom, methyl group, ethyl group, isopropyl group, phenyl group, chlorine atom or bromine atom with the proviso that the substituents R' may be the same of different from one another and m is a number with an average value of from 0 to 5.

## Patentansprüche (Patentansprüche für folgende(n) Vertragsstaat(en): BE, CH, DE, FR, GB, IT, LI, NL)

1. Glycidyletherharz, erhalten aus dem Kondensationsprodukt eines Addukts aus einer ein- oder zweiwertigen Phenolverbindung und einem cyclischen Terpen mit einem Aldehyd oder Keton durch Umsetzung mit einem Epihalogenhydrin.

2. Glycidyletherharz nach Anspruch 1, bei dem das Epihalogenhydrin Epichlorhydrin ist.

3. Hitzehärtbare Harzzusammensetzung, die als Härtungsmittel ein Kondensationsprodukt eines Addukts aus einer ein- oder zweiwertigen Phenolverbindung und einem cyclischen Terpen mit einem Aldehyd oder Keton sowie einen Diglycidylether einer Bishydroxybiphenylverbindung oder ein Polyglycidyletherderivat eines Harzes vom o-Cresol-Novolak-Typ enthält.

4. Zusammensetzung nach Anspruch 3, bei der der Diglycidylether die Formel 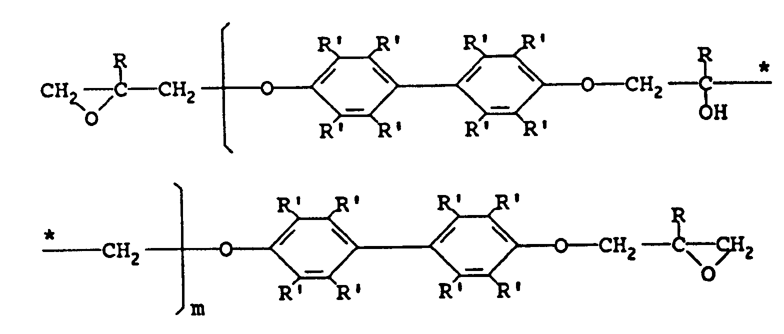 besitzt, bei der R für ein Wasserstoffatom oder eine Methylgruppe, R' für ein Wasserstoffatom, eine Methylgruppe, Ethylgruppe, Isopropylgruppe, Phenylgruppe, ein Chloratom oder ein Bromatom steht, wobei die Substituenten R' gleich oder verschieden sein können und m eine Zahl mit einem Mittelwert von 0 bis 5 ist.

## Patentansprüche (Patentansprüche für folgende(n) Vertragsstaat(en): ES)

1. Verfahren zur Herstellung eines Glycidyletherharzes, bei dem man das Kondensationsprodukt eines Addukts aus einer ein- oder zweiwertigen Phenolverbindung und einem cyclischen Terpen mit einem Aldehyd oder Keton mit einem Epihalogenhydrin umsetzt.

2. Verfahren nach Anspruch 1, bei dem das Epihalogenhydrin Epichlorhydrin ist.

3. Verfahren zur Herstellung einer hitzehärtbaren Harzzusammensetzung, bei dem man ein Kondensationsprodukt eines Addukts aus einer ein- oder zweiwertigen Phenolverbindung und einem cyclischen Terpen mit einem Aldehyd oder Keton mit einem Diglycidylether einer Bishydroxybiphenylverbindung oder mit einem Polyglycidyletherderivat eines Harzes vom o-Cresol-Novolak-Typ vermischt.

4. Verfahren nach Anspruch 3, bei dem der Diglycidylether die Formel 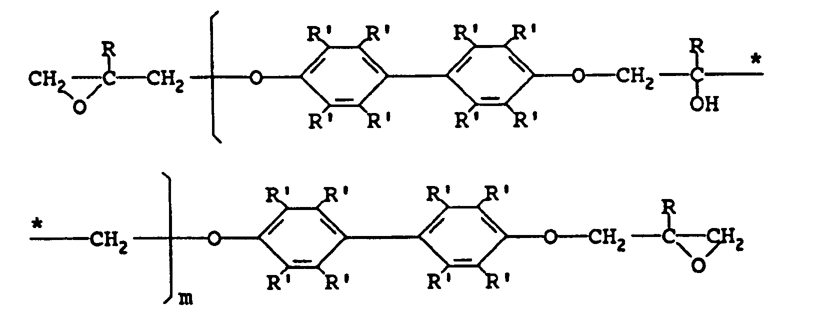 besitzt, bei der R für ein Wasserstoffatom oder eine Methylgruppe, R' für ein Wasserstoffatom, eine Methylgruppe, Ethylgruppe, Isopropylgruppe, Phenylgruppe, ein Chloratom oder ein Bromatom steht, wobei die Substituenten R' gleich oder verschieden sein können und m eine Zahl mit einem Mittelwert von 0 bis 5 ist.

## Revendications (Revendications pour l'(les) Etat(s) contractant(s) suivant(s): BE, CH, DE, FR, GB, IT, LI, NL)

1. Résine d'éther glycidylique obtenue par la réaction d'un produit obtenu en condensant un adduit d'un composé phénolique monohydroxylé ou dihydroxylé et un terpène cyclique et un aldéhyde ou une cétone, avec une épihalohydrine.

2. Résine d'éther glycidylique suivant la revendication 1, caractérisée en ce que l'épihalohydrine est l'épichlorhydrine.

3. Composition de résine thermodurcissable comprenant, à titre d'agent de durcissement, un produit de réaction obtenu en condensant un adduit d'un composé phénolique monohydroxylé ou dihydroxylé et un terpène cyclique avec un aldéhyde ou une cétone, et un éther diglycidylique d'un composé bishydroxybisphénylique, ou un dérivé d'éther polyglycidylique d'une résine du type o-crésol-novolaque.

4. Composition suivant la revendication 3, caractérisée en ce que l'éther diglycidylique répond à la formule : 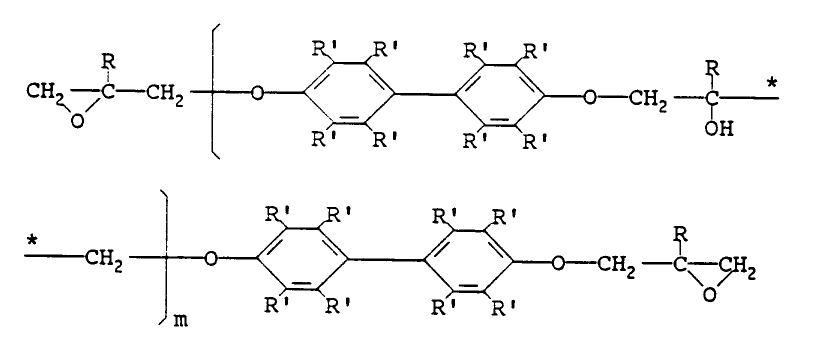 dans laquelle R représente un atome d'hydrogène ou le radical méthyle, R' représente un atome d'hydrogène, le radical méthyle, le radical éthyle, le radical isopropyle, le radical phényle, un atome de chlore ou un atome de brome, avec la condition que les substituants R' peuvent être identiques ou différer les uns des autres et m est un nombre dont la valeur moyenne varie de 0 à 5.

## Revendications (Revendications pour l'(les) Etat(s) contractant(s) suivant(s): ES)

1. Procédé de préparation d'une résine d'éther glycidylique en faisant réagir un produit obtenu en condensant un adduit d'un composé phénolique monohydroxylé ou dihydroxylé et un terpène cyclique et un aldéhyde ou une cétone, avec une épihalohydrine.

2. Procédé suivant la revendication 1, caractérisé en ce que l'épihalohydrine est l'épichlorhydrine.

3. Procédé de préparation d'une composition de résine thermodurcissable en mélangeant mutuellement un produit de réaction obtenu en condensant un adduit d'un composé phénolique monohydroxylé ou dihydroxylé et un terpène cyclique et un aldéhyde ou une cétone, et un éther diglycidylique d'un composé bishydroxybiphénylique, ou un dérivé d'éther polyglycidylique d'une résine du type o-crésol-novolaque.

4. Procédé suivant la revendication 3, caractérisé en ce que l'éther diglycidylique répond à la formule : 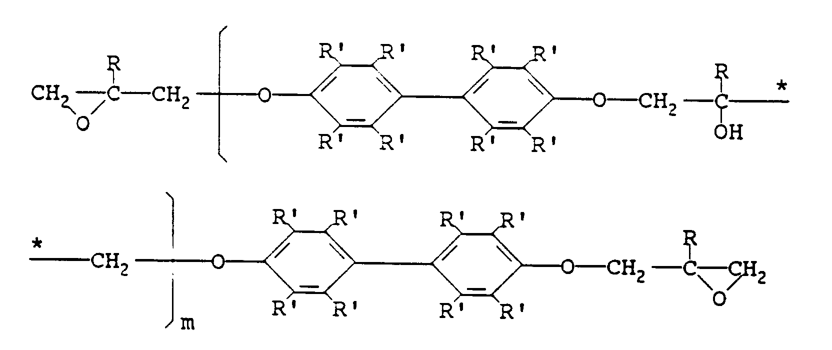 dans laquelle R représente un atome d'hydrogène ou le radical méthyle, R' représente un atome d'hydrogène, le radical méthyle, le radical éthyle, le radical isopropyle, le radical phényle, un atome de chlore ou un atome de brome, avec la condition que les substituants R' peuvent être identiques ou différer les uns des autres et m est un nombre dont la valeur moyenne varie de 0 à 5.
